# FASCICULE DE BREVET EUROPEEN

(11) **EP 2 442 745 B1**
(45) Date de publication et mention de la délivrance du brevet: **30.04.2014**
(21) Numéro de dépôt: 10734509.2
(22) Date de dépôt: 16.06.2010
(51) Int. Cl.: A61B 19/02, A61C 19/02, A61L 2/26, B25H 3/00

(54) **SYSTEME DE RANGEMENT D'INSTRUMENTS**
SYSTEM ZUR AUFBEWAHRUNG VON INSTRUMENTEN
SYSTEM FOR STORING INSTRUMENTS

(30) Priorité: 16.06.2009 FR 0954048
(43) Date de publication de la demande: 25.04.2012
(73) Titulaire: Lachaud, Eric, 78610 Le Perray en Yvelines (FR)
(72) Inventeur: Lachaud, Eric, 78610 Le Perray en Yvelines (FR)
(74) Mandataire: den Braber, Gerard Paul
(86) Numéro de dépôt international: PCT/FR2010/051194
(87) Numéro de publication internationale: WO 2010/146304

(56) Documents cités:
- DE-U1- 8 628 832
- US-A- 1 965 032
- US-A- 5 829 590
- US-A- 5 843 388

## Description

### DOMAINE TECHNIQUE

Un aspect de l'invention concerne un système de rangement d'instruments. Le système peut être utilisé, par exemple, pour ranger des instruments médicaux à stériliser, et notamment des instruments utilisés par des chirurgiens dentistes. Un autre aspect de l'invention concerne un procédé pour stériliser des instruments.

### ETAT DE LA TECHNIQUE ANTERIEURE

Des instruments médicaux pour réaliser un acte donné son typiquement rangés dans plusieurs supports en fonction de leur dimensions respectives. Par exemple, un acte chirurgical dentaire peut impliquer l'utilisation de 4 supports différents. Cela complique la réalisation de l'acte et contribue au risque d'erreurs. En outre, il y a le risque d'une contamination croisée : la transmission de germes d'un patient à un autre par l'intermédiaire d'un praticien. Un geste comme l'ouverture d'un tiroir pour chercher un instrument manquant peut être à l'origine d'une contamination croisée.

Les instruments médicaux, et notamment des instruments de chirurgie dentaire, sont typiquement stérilisés en vrac. Ceci implique un nombre de manipulations relativement important lors d'un processus de stérilisation ; les instruments doivent être manipulés un par un. Il est relativement difficile et laborieux d'effectuer un bon contrôle visuel pour vérifier, par exemple, si une phase de lavage était suffisamment efficace. De plus, il y a un risque non négligeable de blessures et de contamination d'une personne manipulant les instruments lors du processus de stérilisation.

La publication brevet US 5 829 590 décrit une boîte pouvant retenir une pluralité d'instruments dentaires. La boîte comprend des paroirs ouvertes permettent une stérilisation des instruments dentaires dans la boîte en plaçant la boîte dans un environnement de stérilisation comme l'intérieur d'un autoclave.

### EXPOSE DE L'INVENTION

Il existe un besoin pour une solution relativement peu onéreuse permettant de ranger et de stériliser des instruments d'une façon efficace, notamment dans le domaine médical.

Les revendications indépendantes définissent différentes aspect de l'invention. Les revendications dépendantes définissent des caractéristiques supplémentaires qui peuvent être avantageusement appliquées pour mettre en oeuvre l'invention.

Un système de rangement d'instruments conforme à l'invention permet de ranger une grande variété d'instruments de dimensions différentes, notamment en termes de longueur et largeur. Un utilisateur peut donc rationaliser son organisation en regroupant dans un seul système de rangement tous les instruments potentiellement nécessaires pour réaliser un acte donné. Cela facilite la réalisation de l'acte et diminue le risque d'erreurs. En outre, cela diminue le risque d'une contamination croisée : la transmission de germes d'un patient à un autre par l'intermédiaire d'un praticien. Un geste comme l'ouvertured'un tiroir pour chercher un instrument peut être à l'origine d'une contamination croisée. De tels gestes peuvent être évités en regroupant tous les instruments nécessaires dans un seul système de rangement.

Un système de rangement d'instruments conforme à l'invention permet de stériliser des instruments rangés dedans de façon adéquate et efficace. Plusieurs facteurs contribuent à ceci. Le système de rangement d'instruments permet un bon contrôle visuel pour vérifier, par exemple, si une phase de lavage était suffisamment efficace. Le système de rangement d'instruments peut être implémenté de façon à ce que ces éléments constitutifs soient légèrement mobiles entre eux et ne possèdent pas de grandes surfaces de contact susceptibles de retenir des saletés, comme par exemple, des mucosités, du sang, et de la salive. Il en va de même pour les contacts entre les instruments et le système de rangement d'instruments. Ces contacts sont lâches et permettent aux instruments de bouger au cours du processus de stérilisation. En outre, le système de rangement d'instruments est relativement facile à manipuler au cours des différentes phases de stérilisation. Relativement peu de manipulations sont requises pour stériliser une quantité d'instruments donnée. Normalement aucun instrument ne peut tomber. Ceci limite le risque de blessures et de contamination du personnel au sein du centre de stérilisation.

Une description détaillée en référence à des dessins illustre l'invention brièvement exposée précédemment, ainsi que les caractéristiques supplémentaires identifiées précédemment.

### DESCRIPTION SOMMAIRE DES DESSINS

- La figure 1 est un diagramme pictural illustrant un système de rangement d'instruments.
- La figure 2 est un diagramme schématique illustrant une vue de face du système de rangement d'instruments.
- La figure 3 est un diagramme schématique illustrant une vue de dessus du système de rangement d'instruments.
- La figure 4 est un diagramme schématique illustrant une vue de coté du système de rangement d'instruments.
- La figure 5 est un organigramme illustrant un procédé pour stériliser des instruments.

### DESCRIPTION DETAILLEE

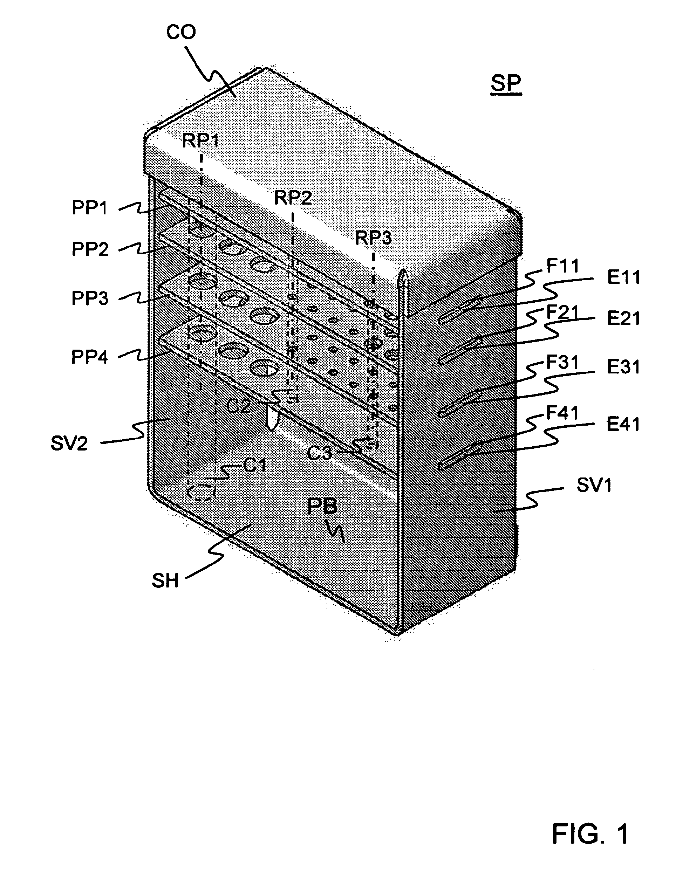

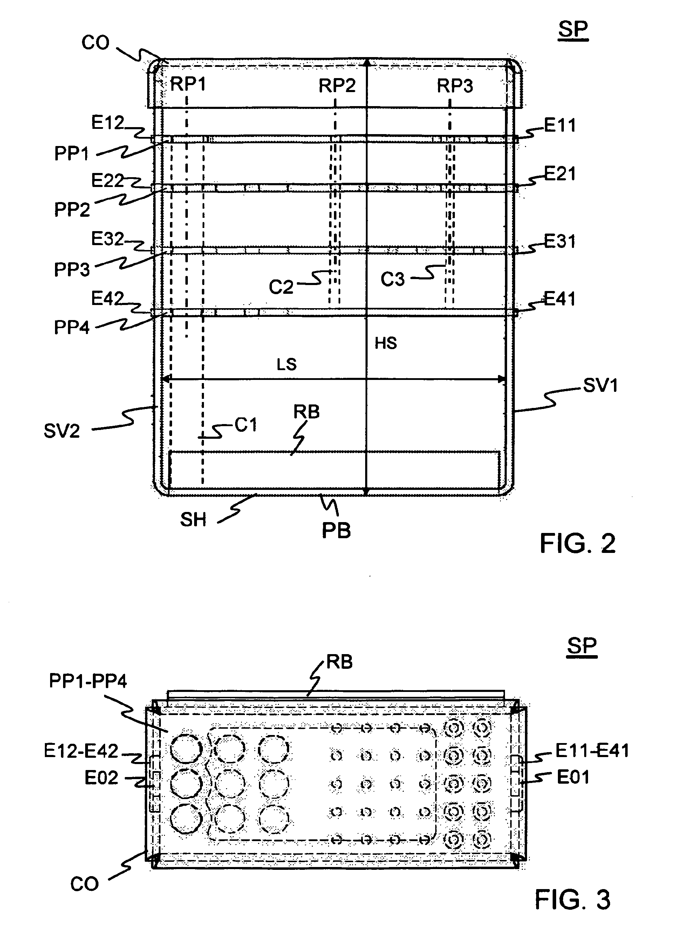

Les figures 1-4 illustrent un exemple d'un système de rangement d'instruments SP, chacune de façon différente. La figure 1 illustre le système de rangement d'instruments SP de façon picturale, comme si une photo de celui-ci avait été prise. Les figures 2-4 illustrent le système de rangement d'instruments SP de façon schématique respectivement par une vue de face, une vue de dessus, et une vue de côté.

Le système de rangement d'instruments SP illustré aux figures 1-4 comprend une pièce de base PB avec un segment plan horizontal SH et deux segments plans verticaux SV1, SV2 disposés sous forme d'un U. En position normale, qui permet de ranger un instrument ou de saisir un instrument, le segment plan horizontal SH est posé sur une surface stable comme, par exemple, un plan de travail. Le segment plan horizontal SH comprend un rebord RB, ce qui contribue à la stabilité et la rigidité de la pièce de base PB et donc du système de rangement d'instruments SP. La pièce de base PB peut être formée, par exemple, d'une plaque de métal inoxydable pliée en U. Cette plaque peut être, par exemple, en inox de type 304L et avoir une épaisseur de 1 millimètre (mm).

Le système de rangement d'instruments SP comprend une pluralité de platines perforées PP1-PP4 disposées entre les deux segments plans verticaux SV1, SV2. Les platines perforées PP1-PP4 sont disposées de façon parallèle par rapport au segment plan horizontal SH de la pièce de base PB. Les platines perforées PP1-PP4 sont agencées de façon à ce qu'il existe plusieurs rangées de perforations alignées verticalement. C'est-à-dire, une rangée de perforations comprend une pluralité de perforations dont chacune appartient à une platine perforée différente.

Les figures 1 et 2 indiquent trois rangées de perforations RP1, RP2, RP3. Chaque rangée comprend au moins deux perforations qui sont disposées coaxialement entre elles. C'est-à-dire, pour chaque rangée, il existe un axe vertical sur lequel au moins deux perforations sont centrées. Sur les figures 1 et 2, cet axe est indiqué au moyen d'une ligne tiret-point pour les rangées de perforations RP1, RP2, RP3.

La rangée de perforations RP1 définit une colonne C1 qui s'étend de la platine perforée PP1, qui est la platine la plus éloignée du segment plan horizontal SH, jusqu'au segment plan horizontal SH. Cette colonne C1 a donc une longueur qui correspond à la distance entre la platine perforée RP1 et le segment plan horizontal SH. Cette longueur caractérise la rangée de perforations RP1. La rangée de perforations RP1 est également caractérisée par les dimensions respectives des perforations respectives appartenant à la rangée RP1. Ces caractéristiques permettent à la rangée de perforations RP1 d'accueillir un instrument spécifique ayant des dimensions spécifiques. En effet, une partie substantielle de l'instrument sera comprise dans la colonne C1 indiquée à la figure 1.

La rangée de perforations RP2 définit une colonne C2. Cette colonne C2 s'étend de la platine perforée PP1 jusqu'à la platine perforée PP14. Partant de la platine PP1, en allant en direction du segment plan horizontal SH, la platine perforée PP4 est la première platine qui n'a pas de perforations appartenant à la rangée RP2. La colonne C2 a donc une longueur qui correspond à la distance entre la platine perforée PP1 et la platine perforée PP4. Cette longueur est inférieure à la longueur de la colonne C1 définie par la rangée de perforations RP1. En outre, les perforations appartenant à la rangée RP2 ont des dimensions qui sont inférieures à celles des perforations appartenant à la rangée RP1. La rangée de perforations RP2 est donc agencée pour accueillir un instrument spécifique qui est relativement petit par rapport à l'instrument que la rangée RP1 peut accueillir, notamment en termes de longueur et de la largeur. Il en va de même pour la rangée de perforations RP3 qui définit une colonne C3.

En règle générale, une rangée de perforations définit une colonne qui s'étend de la platine perforée PP1, qui est la plus éloignée du segment plan horizontal SH, en direction de celui-ci jusqu'à une platine n'ayant pas de perforations appartenant à la rangée ou, dans le cas où toutes les platines ont des perforations appartenant à la rangée, jusqu'au segment plan horizontal SH. La colonne a une longueur spécifique. Les perforations de la rangée ont des dimensions spécifiques. Cette longueur spécifique et ses dimensions spécifiques permettent à la rangée de perforation d'accueillir un instrument spécifique.

Une rangée de perforations permet donc de laisser passer ou non un instrument en fonction de ses dimensions, notamment en termes de longueur et de largeur. Un instrument émerge suffisamment de la platine perforée PP1, qui est la plus éloignée du segment plan horizontal SH, pour qu'un utilisateur puisse s'en saisir aisément avec une paire de précelles.

Le segment plan vertical SV1 comprend une pluralité de fentes F11-F41 illustrée à la figure 1. Le segment plan vertical SV2 comprend également une pluralité de fentes disposées symétriquement par rapport à celles dans le segment plan vertical SV1. Les fentes dans le segment plan vertical SV2 ne sont pas indiquées aux figures 1-4 pour des raisons de concision.

En d'autres termes, les deux segments plans verticaux SV1, SV2 comprennent une pluralité de paires de fentes. Chaque paire comprend une fente dans le segment plan vertical SV1 et une autre fente dans le segment plan vertical SV2. Chaque paire de fentes est disposée dans un plan parallèle au segment plan horizontal SH.

La figure 2 illustre que chaque platine perforée est munie d'une paire d'ergots s'encastrant dans une paire de fentes dans les deux segments plans verticaux SV1, SV2. Par exemple, la figure 2 illustre une paire d'ergots E11-E12 appartenant à la platine perforée PP1 avec un ergot E11 s'encastrant dans une fente dans le segment plan vertical SV1, l'autre ergot E12 s'encastrant dans une fente dans le segment en vertical SV2. La figure 1 illustre des ergots E11-E41 respectifs appartenant aux platines perforées PP1-PP4 respectives qui sont encastrées dans les fentes F11-F41 respectives dans le segment plan vertical SV1. Chacune des fentes peut être formée d'une rangée de perforations dans laquelle deux perforations voisines se chevauchent.

Le système de rangement d'instruments SP comprend un couvercle amovible CO pouvant s'engager avec les deux segments plans verticaux SV1, SV2 de la pièce de base PB. La figure 1 illustre que le couvercle amovible CO est rectangulaire et présente 4 côtés. Chaque côté possède un repli. Deux replis enserrent les deux segments plans verticaux SV1, SV2. Ainsi le couvercle amovible CO empêche un écartement des segments plans verticaux SV1, SV2 ce qui pourrait provoquer une chute des platines perforées PP1-PP4. Afin de verrouiller le couvercle amovible CO sur la pièce de base PB, les deux replis enserrant les deux segments plans verticaux SV1, SV2 comprennent des parties embossées EO1, E02 qui sont représentés à la figure 3. Les deux segments plans verticaux SV1, SV2 comprennent dans une partie supérieure avec des perforations dans lesquelles ces parties embossées EO1, EO2 peuvent se loger.

Sans la présence du couvercle amovible CO, les deux segments plans verticaux SV1, SV2 se laissent écarter entre eux de façon élastique. Ainsi, en enlevant le couvercle amovible CO, il est possible de remplacer une ou plusieurs platines perforées illustrées à la figure 1 par d'autres platines. La pièce de base PB est conçue de telle façon qu'en situation de repos les platines perforées PP1-PP4 sont retenues par les deux segments plans verticaux SV1, SV2. En posant le couvercle amovible CO sur la pièce de base PB, cette situation est maintenue même si le système de rangement d'instruments SP subi des manipulations, par exemple, au cours d'un processus de stérilisation.

Le système de rangement d'instruments SP est donc modulaire car les platines perforées PP1-PP4 sont amovibles. Le système de rangement d'instruments SP peut donc comprendre de nombreux différents jeux de platines perforées. Un jeu de platines perforées particulier fournit une pluralité de rangée de perforations pouvant accueillir un jeu d'instruments particulier.

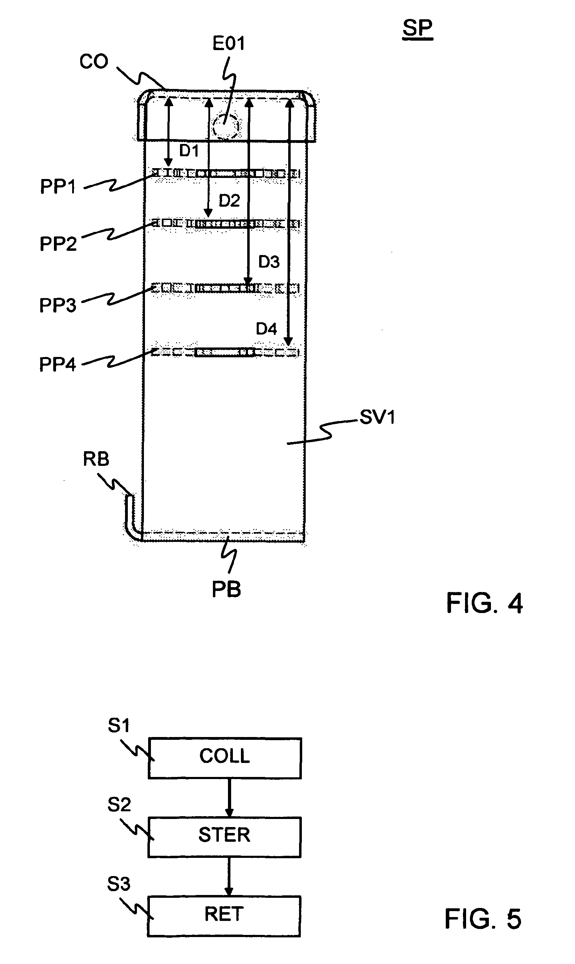

La figure 4 illustre que les platines perforées PP1-PP4 sont positionnées à des distances respectives D1-D4 du couvercle amovible CO quand celui-ci est posé sur la pièce de base. En d'autres termes, les platines perforées PP1-PP4 sont positionnées à des distances respectives D1-D4 du haut des segments plans verticaux SV1, SV2. Ces distances D1-D4 sont principalement déterminées par les instruments à ranger dans le support de rangement d'instruments SP. C'est-à-dire, les distances D1-D4 peuvent varier en fonction d'un ou plusieurs jeux d'instruments à ranger.

Grâce à son caractère modulaire, le système de rangement d'instruments SP permet de ranger une grande variété d'instruments de dimensions différentes, notamment en termes de longueur et largeur. Un utilisateur peut donc rationaliser son organisation en regroupant dans un seul système de rangement tous les instruments potentiellement nécessaires pour réaliser un acte donné. Cela facilite la réalisation de l'acte et diminue le risque d'erreurs. En outre, cela diminue le risque d'une contamination croisée : la transmission de germes d'un patient à l'autre un autre par l'intermédiaire d'un praticien. Un geste comme l'ouverture d'un tiroir pour chercher un instrument peut être à l'origine d'une contamination croisée. De tels gestes peuvent être évités en regroupant tous les instruments nécessaires dans un seul système de rangement.

Le système de rangement d'instruments SP est particulièrement adapté pour une utilisation en chirurgie dentaire. Le système de rangement d'instruments SP peut accueillir tout type de petits instruments comme, par exemple, des fraises et des forêts pour des instruments rotatifs, des petits instruments manuels, et des inserts à ultrasons. Se référant à la figure 2, dans une telle utilisation, le système de rangement d'instruments SP peut avoir, par exemple, une hauteur HS et une largeur LS de l'ordre de quelques dizaines de millimètres jusqu'à quelques centaines de millimètres.

Des systèmes de rangement d'instruments tel que décrits dans ce qui précède en référence aux figures 1-4, ainsi que des systèmes de rangement d'instruments similaires, peuvent être placées dans des cassettes pour instruments. Dans ce cas, la hauteur HS illustrée à la figure 2 doit correspondre à une hauteur standard adaptée pour ces cassettes pour instruments. Cette hauteur peut être relativement importante par rapport à la longueur des instruments à ranger dans un système de rangement d'instruments. Dans ce cas, une partie inférieure du système de rangement d'instruments sera disponible. Cette partie inférieure sera comprise entre le segment plan horizontal et la platine perforée la plus proche de celui-ci. La partie intérieure peut être utilisée pour ranger des instruments en munissant le système de rangement d'instruments de deux segments plans additionnels, de préférence perforés, disposés orthogonalement entre le segment plan horizontal et la platine perforée la plus proche de celui-ci. Un de ces deux segments plans additionnels peut être sous forme d'une porte, l'autre peut correspondre à un rebord du segment plan horizontal.

La figure 5 est un organigramme illustrant un procédé pour stériliser des instruments. Ce procédé est basé sur l'utilisation de systèmes de rangement d'instruments tel que décrits dans ce qui précède en référence aux figures 1-4, ou des systèmes de rangement d'instruments similaires.

Dans une première étape S1, des instruments à stériliser sont collectés et transportés vers un centre de stérilisation (COLL). Ces instruments à stériliser sont rangés dans des systèmes de rangement d'instruments tel que décrits dans ce qui précède en référence aux figures 1-4, ainsi que dans des systèmes de rangement d'instruments similaires. Les systèmes de rangement d'instruments peuvent se trouver à différentes adresses, par exemple, à différents cabinets dentaires. Dans ce cas, un véhicule passe auprès de ces différentes adresses pour récolter les systèmes de rangement d'instruments contenant des instruments à stériliser pour les transporter au centre de stérilisation. Une cassette pour instruments mentionnée dans ce qui précède permet de regrouper plusieurs systèmes de rangement d'instruments contenant des instruments à stériliser.

Dans une deuxième étape S2, les instruments rangés dans les systèmes de rangement d'instruments sont stérilisés au sein d'un centre de stérilisation (STER). Un processus de stérilisation comprend typiquement les phases suivantes : lavage, séchage, contrôle visuel, et diffusion de vapeur d'eau à la surface des instruments. Pendant ce processus, les instruments sont retenus par les systèmes de rangement d'instruments.

Un système de rangement d'instruments tel que celui décrit dans ce qui précède permet de stériliser des instruments rangés dedans de façon adéquate et efficace. Plusieurs facteurs contribuent à ceci. Le système de rangement d'instruments permet un bon contrôle visuel pour vérifier, par exemple, si la phase de lavage était suffisamment efficace. Grâce à sa conception, les différents éléments constitutifs du système de rangement d'instruments sont tous légèrement mobiles entre eux et ne possèdent pas de grandes surfaces de contact susceptibles de retenir des saletés, comme par exemple, des mucosités, du sang, et de la salive. Il en va de même pour les contacts entre les instruments et le système de rangement d'instruments. Ces contacts sont lâches et permettent aux instruments de bouger au cours du processus de stérilisation. En outre, le système de rangement d'instruments est relativement facile à manipuler au cours des différentes phases de stérilisation. Relativement peu de manipulations sont requises pour stériliser une quantité d'instruments donnée. Normalement aucun instrument ne peut tomber. Ceci limite le risque de blessures et de contamination du personnel au sein du centre de stérilisation.

Dans une troisième étape (S3), les systèmes de rangement d'instruments, et les instruments à l'intérieur de ceux-ci, ayant subi le processus de stérilisation, sont retournés vers leurs utilisateurs respectifs (RET). Un système de rangement d'instruments stérilisé peut être mis en sachet par le centre de stérilisation pour le transport vers son utilisateur.

### REMARQUES FINALES

La description détaillée en référence aux figures est simplement une illustration de l'invention. L'invention peut être réalisée de nombreuse façons différentes. Afin d'illustrer ceci, quelques alternatives sont indiquées sommairement.

Il existe de nombreuses façons d'implémenter un système de rangement d'instruments selon l'invention. Le système de rangement d'instruments SP décrit en référence aux figures 1 à 4 n'est qu'un exemple à titre purement illustratif. Il est possible de réaliser un système de rangement d'instruments adapté pour ranger un quelconque jeu d'instruments. Il suffit de convenablement déterminer les paramètres suivants : nombre de platines perforées, leurs positions respectives en termes de hauteur, le nombre de perforations dans chaque platine, leur positions respectives, et leurs diamètres respectifs. A cet égard il convient de noter que les deux segments plans verticaux peuvent chacun être muni d'une grille de fentes relativement dense et des marqueurs permettant de marquer, pour un jeu d'instruments particulier, des positions préférentielles pour les platines perforées.

Il existe différentes façons pour retenir les platines perforées entre deux segments plans verticaux. L'utilisation de fentes et ergots à cet effet n'est qu'un exemple. Une autre façon pour retenir les platines perforées consiste à, par exemple, munir les deux segments plans verticaux de pinces.

Un système de rangement d'instruments peut comprendre des éléments de couplage pour coupler le système à un autre système de rangement d'instruments. Ainsi, il est possible de réaliser un super-système comprenant une rangée de système de rangement d'instruments solidarisés entre eux.

L'expression « sous forme d'un U » doit être interprétée de façon large. Cette expression n'exclut pas des modes de réalisation comprenant un segment plan horizontal ayant des dimensions supérieures par rapport aux deux segments plans verticaux.

Les remarques qui précèdent montrent que la description détaillée en référence aux figures, illustre l'invention plutôt qu'elle ne la limite. Les signes de références n'ont aucun caractère limitatif.

## Revendications

1. Système de rangement d'instruments (SP) comprenant :
- une pièce de base (PB) comprenant un segment plan horizontal (SH) et deux segments plans verticaux (SV1, SV2) disposés sous forme d'un U ; et
- une pluralité de platines perforées (PP1-PP4) disposées entre les deux segments plans verticaux (SV1, SV2) et de façon parallèle par rapport au segment plan horizontal (SH) de la pièce de base (PB), les platines perforées étant agencées de façon à ce qu'il existe plusieurs rangées de perforations (RP1, RP2, RP3) alignées verticalement, une rangée de perforations (RP1) définissant une colonne (C1) s'étendant de la platine perforée (PP1) la plus éloignée du segment plan horizontal (SH) en direction de celui-ci jusqu'à une platine perforée n'ayant pas de perforations appartenant à la rangée ou, dans le cas où toutes les platines ont des perforations appartenant à la rangée, jusqu'au segment plan horizontal (SH), la colonne ayant une longueur spécifique, et les perforations de la rangée ayant des dimensions spécifiques, afin que la rangée de perforations puisse accueillir un instrument spécifique,
- **caractérisé en ce que** les platines perforées sont amovibles, les deux segments plans verticaux (SV1, SV2) comprenant une pluralité d'agencements de rétention (F11-F41), un agencement de rétention étant disposé dans un plan parallèle au segment plan horizontal (SH) et pouvant retenir dans ce plan une platine perforées entre les deux segments plans verticaux (SV1, SV2) de la pièce de base (PB).

2. Système de rangement d'instruments selon la revendication 1, dans lequel les agencements de rétention comprennent des fentes (F11-F41), les platines perforées comprenant des ergots (E11-E41) pouvant s'encastrer dans ces fentes, la pièce de base (PB) étant conçue de telle façon que ces deux segments plans verticaux (SV1, SV2) se laissent écarter entre eux de façon élastique.

3. Système de rangement d'instruments selon la revendication 2, dans lequel une fente est formée d'une rangée de perforations dans laquelle deux perforations voisines se chevauchent.

4. Système de rangement d'instruments selon l'une quelconque des revendications précédentes, dans lequel le système de rangement d'instruments (SP) comprend un couvercle amovible (CO) pouvant s'engager avec les deux segments plans verticaux (SV1, SV2) de la pièce de base (PB).

5. Système de rangement d'instruments selon la revendication 4, dans lequel le couvercle amovible (CO) comprend des parties embossées pouvant se loger dans des perforations se trouvant dans une partie supérieure des deux segments plants verticaux (SV1, SV2).

6. Système de rangement d'instruments selon l'une quelconque des revendications précédentes, dans lequel la pièce de base (PB) comprend un rebord (RB) solidaire au segment plan horizontal (SH) et orthogonal à celui-ci.

7. Système de rangement d'instruments selon l'une quelconque des revendications précédentes, dans lequel la pièce de base (PB) est formée d'une plaque pliée en U.

8. Système de rangement d'instruments selon l'une quelconque des revendications précédentes, dans lequel la pièce de base (PB) est composée d'un métal inoxydable.

9. Procédé pour stériliser des instruments, comprenant une étape (S2) consistant à appliquer un processus de stérilisation à un système de rangement d'instruments (SP) selon l'une quelconque des revendications précédentes, le système de rangement d'instruments comprenant des instruments à stériliser.

## Patentansprüche

1. Aufbewahrungssystem für Instrumente (SP) umfassend:
- eines Basiselements (PB) mit einem flächigen horizontalen Abschnitt (SH) und zwei flächigen vertikalen Abschnitten (SV1, SV2) in U-förmiger Anordnung; und
- eine Vielzahl von perforierter Platinen (PP1-PP4), die zwischen den zwei flächigen vertikalen Abschnitten (SV1, SV2) angebracht sind und parallel zum flächigen horizontalen Abschnitt (SH) des Basiselements (PB), wobei die perforierten Platinen so angeordnet sind, dass mehrere Reihen von Perforationen (RP1, RP2, RP3) in vertikaler Ausrichtung existieren, eine Reihe von Perforationen (RP1) bildet eine Spalte (C1), die sich von der perforierten Platine (PP1), die sich am weitesten vom flächigen horizontalen Abschnitt (SH) entfernt befindet in dessen Richtung erstreckt bis zu einer perforierten Platine, die keine zu der Reihe gehörigen Perforationen hat oder, falls alle Platinen zu der Reihe gehörige Perforationen haben, bis zum flächigen horizontalen Abschnitt (SH), wobei die Spalte eine spezifische Länge hat und die Perforationen der Reihe spezifische Abmessungen haben, damit die Reihe von Perforationen ein spezifisches Instrument aufnehmen kann,
- **gekennzeichnet dadurch, dass** die perforierten Platinen abnehmbar sind, die beiden flächigen vertikalen Abschnitte (SV1, SV2) eine Vielzahl von Rückhalteanordnungen (F11-F41) beinhalten, eine Rückhalteanordnung in einer parallelen Fläche zum flächigen horizontalen Abschnitt (SH) angebracht ist und auf dieser Fläche eine perforierte Platine zwischen den zwei vertikalen flächigen Abschnitten (SV1, SV2) des Basiselements (PB) zurückhalten kann.

2. Aufbewahrungssystem für Instrumente gemäß Anspruch 1, in dem die Rückhalteanordnungen Schlitze aufweisen (F11-F41), die perforierten Platinen mit Rastnasen ausgestattet sind (E11 -E41), die in diese Schlitze einrasten können, das Basiselement (PB) so gestaltet ist, dass diese beiden vertikalen flächigen Abschnitte (SV1, SV2) sich untereinander elastisch auseinander bewegen lassen.

3. Aufbewahrungssystem für Instrumente gemäß Anspruch 2, in dem ein Schlitz aus einer Reihe von Perforationen gebildet wird, in der sich zwei benachbarte Perforationen überlagern.

4. Aufbewahrungssystem für Instrumente gemäß einem beliebigen der vorstehenden Ansprüche, in dem das Aufbewahrungssystem für Instrumente (SP) eine abnehmbare Abdeckung (CO) beinhaltet, die mit den beiden vertikalen flächigen Abschnitten (SV1, SV2) des Basiselements (PB) verbunden werden kann.

5. Aufbewahrungssystem für Instrumente gemäß Anspruch 4, in dem die abnehmbare Abdeckung (CO) geprägte Teile enthält, die in den Perforationen in einem oberen Teil der beiden vertikalen flächigen Abschnitten (SV1, SV2) untergebracht werden können.

6. Aufbewahrungssystem für Instrumente gemäß einem beliebigen der vorstehenden Ansprüche, in dem das Basiselement (PB) einen Rand (RB) umfasst, der mit dem flächigen horizontalen Abschnitt (SH) verbunden ist und im rechten Winkel zu diesem steht.

7. Aufbewahrungssystem für Instrumente gemäß einem beliebigen der vorstehenden Ansprüche, in dem das Basiselement (PB) aus einer U-förmig gebogenen Platte gebildet ist.

8. Aufbewahrungssystem für Instrumente gemäß einem beliebigen der vorstehenden Ansprüche, in dem das Basiselement (PB) aus rostfreiem Metall besteht.

9. Verfahren zur Sterilisierung der Instrumente, inklusive einer Etappe (S2), die darin besteht, einen Sterilisierungsvorgang an einem Aufbewahrungssystem für Instrumente (SP) nach einem beliebigen der vorstehenden Ansprüche anzuwenden, wobei das Aufbewahrungssystem für Instrumente zu sterilisierende Instrumente enthält.

## Claims

1. Instrument storage system (SP) comprising:
- a base part (PB) comprising a plane horizontal segment (SH) and two plane vertical segments (SV1, SV2) arranged in U-shape; and
- a plurality of perforated plates (PP1-PP4) arranged between the two plane vertical segments (SV1, SV2) and parallel with respect to the plane horizontal segment (SH) of the base part (PB), the perforated plates being designed so that there are several rows of vertically aligned perforations (RP1, RP2, RP3), a row of perforations (RP1) defining a column (C1) extending from the perforated plate (PP1) farthest from the plane horizontal segment (SH) towards it up to a perforated plate with no perforations belonging to the row or, if all the plates have perforations belonging to the row, up to the plane horizontal segment (SH), the column having a specific length, and the perforations of the row having specific dimensions, so that the row of perforations can accommodate a specific instrument,
- **characterised in that** the perforated plates are removable, the two plane vertical segments (SV1, SV2) comprising a plurality of retention fittings (F11-F41), a retention fitting being arranged in a plane parallel to the plane horizontal segment (SH) and being able to retain in this plane a perforated plate between the two plane vertical segments (SV1, SV2) of the base part (PB).

2. Instrument storage system according to claim 1, wherein the retention fittings comprise slots (F11-F41), the perforated plates comprising lugs (E11 -E41) that can fit in these slots, the base part (PB) being designed so that these two plane vertical segments {SV1, SV2) can move away from each other elastically.

3. Instrument storage system according to claim 2, wherein a slot is formed by a row of perforations in which two adjacent perforations overlap.

4. Instrument storage system according to any of the preceding claims, wherein the instrument storage system (SP) comprises a removable cover (CO) that can engage with the two plane vertical segments (SV1, SV2) of the base part (PB).

5. Instrument storage system according to claim 4, wherein the removable cover (CO) comprises embossed parts that can fit in the perforations in an upper part of the two plane vertical segments (SV1, SV2).

6. Instrument storage system according to any of the preceding claims, wherein the base part (PB) comprises an edge (RB) attached to the plane horizontal segment (SH) and perpendicular to it.

7. Instrument storage system according to any of the preceding claims, wherein the base part (PB) is formed from a plate folded into a U-shape.

8. Instrument storage system according to any of the preceding claims, wherein the base part (PB) is composed of a stainless metal.

9. Method for sterilising instruments, comprising a step (S2) which consists in applying a sterilisation process to an instrument storage system (SP) according to any of the preceding claims, the instrument storage system comprising instruments to be sterilised.
